# EUROPEAN PATENT APPLICATION

(11) **EP 1 413 300 A1**
(43) Date of publication of application: **28.04.2004**
(21) Application number: 02024004.0
(22) Date of filing: 26.10.2002
(51) Int. Cl.: A61K 31/155, A61K 31/505, A61K 31/506, C07D 239/48, C07C 281/18, C07D 403/12, C07D 257/06, A61P 31/18, A61P 31/22, A61P 31/12, A61P 37/06, A61P 19/02, A61P 35/00

(54) **Pyrimidinium-guanidiniminoethylphenyl-guanidinoalkyl-amines and guanidiniminoethylphenyl-guanidinoalkylamines as pharmaceutically active new substances**

(71) Applicant: Mondobiotech SA, 6925 Gentilino (CH)
(72) Inventor: Bevec, Dorian, Dr., 6925 Gentilino (CH)
(74) Representative: Salgo, Reinhold Caspar, Dr.

(57) **Abstract**

The present invention relates to new compositions and methods for use as pharmaceutically active agents, especially for prophylaxis and prevention in virally caused infections and diseases, tumor necrosis factor alpha (TNFα)-related inflammatory conditions and diseases, graft rejection after organ or tissue transplantations, and cancer. The innovative compounds are inhibitors of Hypusine formation and nuclear transport of distinct RNAs from nucleus to cytoplasm.

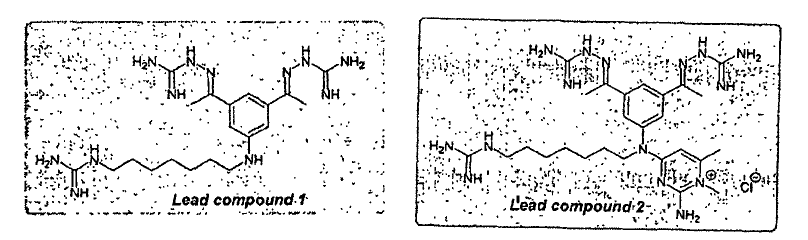

## Description

The present invention relates to new compositions and methods that are useful in preventing and ameliorating
1) virally caused infections and diseases;
2) tumor necrosis factor alpha (TNFα)-related inflammatory conditions and diseases;
3) graft rejection after organ or tissue transplantations;
4) cancer.

More particularly, the invention relates to novel chemical entities termed 3,5-Bis-(guanidiniminoethylphenyl)-guanidinoheptyl-amines and (2-Amino-1-methyl-pyridiniumchloride)-[3,5-bis-(guanidiniminoethylphenyl]-*N*-guanidinoheptyl-amines, their compositions and their use to inhibit the the formation of the unusual amino acid Hypusine that is formed on the human protein eukaryotic initiation factor 5A (eIF-5A), a protein involved in the nucleo-cytoplasmic translocation of distinct cellular and viral messenger RNAs from the nucleus of a cell to the ribosomes in the cytoplasm, where protein synthesis occurs.
Thus, the novel agents are considered as nuclear transport inhibitors for prevention and treatment of virally induced infections and diseases.

These compositions and methods are also useful in preventing the generation of TNFα protein by cells, and so to prevent TNFα-related inflammatory conditions and diseases.

In another embodiment, the compounds of the present invention can be used to inhibit graft rejections after organ or tissue transplantations as.

The novel compounds of the invention are also useful as inhibitors of the maturation of immunocompetent dendritic cells.

In addition, the novel compounds are useful for treating and preventing cellular hyperproliferation in cancer conditions.

### Description of the invention

Object of the present invention is to provide pharmaceutically active agents together with methods for treating complications associated with viral infections, TNFα-related inflammatory conditions, with rejections of grafts in organ or tissue transplantations and in cancer.
This objects are solved by the disclosure of the independent claims. Further advantageous features, aspects and details of the invention are evident from the dependent claims, the description, and the examples of the present application.

The present invention relates to a compound of the general formula (I): 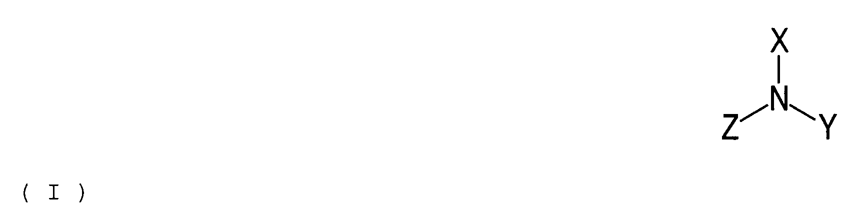 wherein:
X is independently of each other 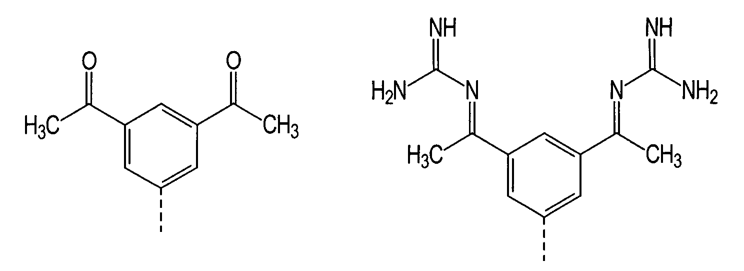
Y is independently of each other 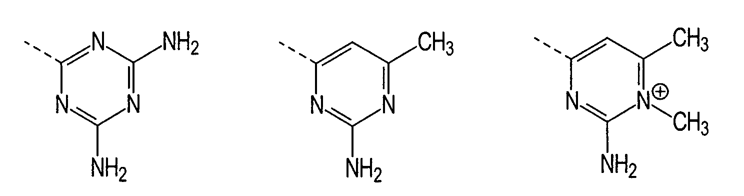
Z is independently of each other 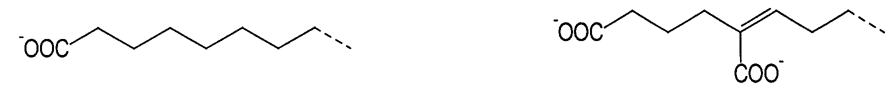 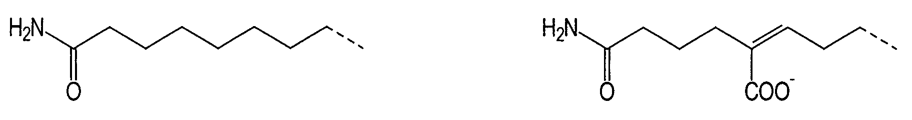 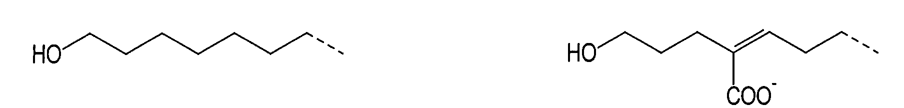 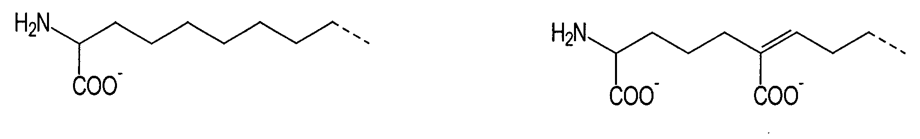
or to a compound selected form the following general formulas (II) 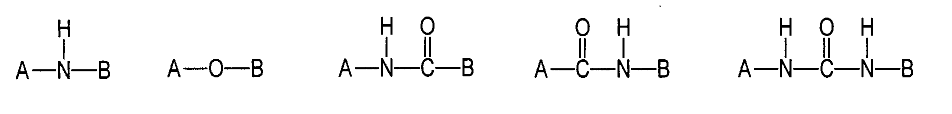 wherein
A is independently of each other 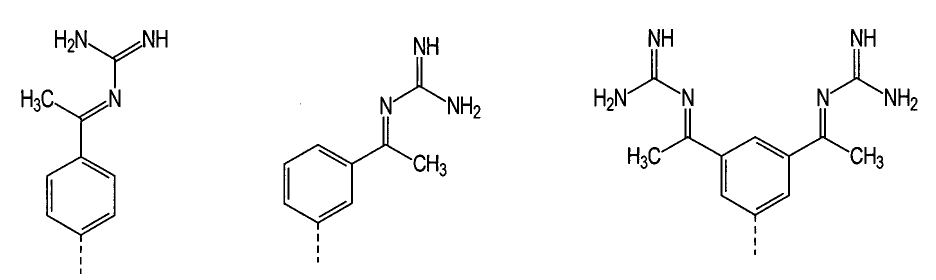 and
B is independently of each other

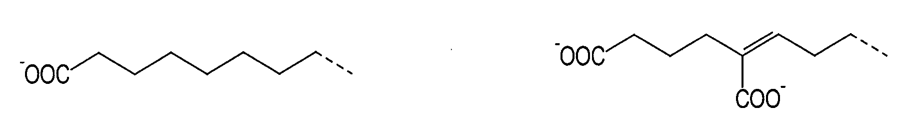

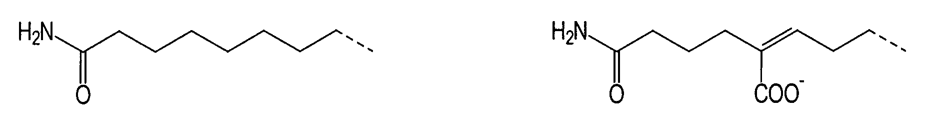

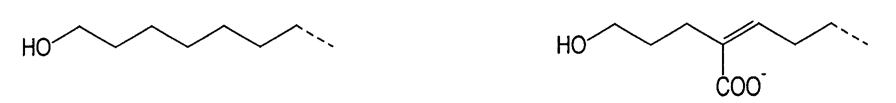

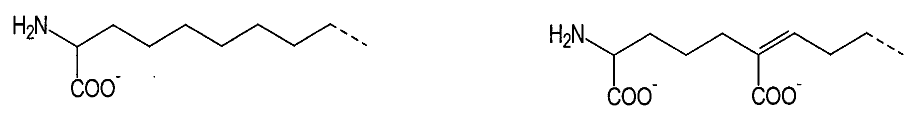

Surprisingly, the inventive compounds exhibit excellent activity against viral infections, inhibit TNFα formation, inhibit maturation of dendritic cells and inhibit proliferation of tumor cells.

Preferred is the use of a compound of formula (I) termed *N*-{7-[3,5-Bis-(1-guanidiniminoethyl)-phenylamino]-heptyl}-guanidine or
*N*-[3,5-Bis-(1-guanidiniminoethyl)-phenyl]-*N*-(7-guanidinoheptyl)-amine or a salt thereof
and/or compound of formula (I) termed *N*-(7-{(2-Amino-1-methylpyridiniumchloride-4-yl)-[3,5-bis-(1-guanidiniminoethyl)-phenylamino]-heptyl}-guanidine or
*N*-(2-Amino-1-methyl-pyridiniumchloride-4-yl)-*N*-[3,5-bis-(1-guanidiniminoethyl)-phenyl]-*N*-(7-guanidinoheptyl)-amine or a salt thereof.

### Background of the invention

### 1) Virally caused infections and diseases

The agents of the invention are beneficial in the treatment of HIV-1, HTLV-I, HBV, HCV, RSV, EBV, HHV8, VZV, or HSV1.

### The Human Immunodeficiency Virus Type I (HIV-1)

HIV is a complex retrovirus encoding in addition to the structural proteins gag, pol, and env several regulatory genes. Specifically, the trans-acting regulator of viral gene expression, called Rev, is crucially important for HIV replication. The HIV Rev protein is a nuclear phosphoprotein accumulating at steady state in the nucleoli and has the capacity to shuttle between the nuclear and cytoplasmic compartment. Within the Rev protein distinct biological domains exist that are essential for its biological activity. The cis-acting target for Rev is a highly structured sequence element termed the Rev Response Element (RRE) located on HIV RNA. Upon binding to the HIV RNA as a monomer, Rev has to multimerize and subsequently interact with cellularly encoded cofactors on its way to export the HIV RNA from the nucleus to the cytoplasm where protein translation occurs. To date, Rev is the most studied specific RNA export factor containing a leucine-rich nuclear export signal (NES). It was shown that the eukaryotic initiaton factor 5A (eIF-5A) directly and specifically binds the HIV Rev NES. Distinct eIF-5A mutants have been shown to inhibit nuclear export of Rev proteins and, thereby, HIV replication. Moreover, anti eIF-5A antibodies specifically block the nucleocytoplasmic translocation of Rev, indicating that eIF-5A is part of a specific nucleocytoplasmic export pathway utilized by HIV. In general, the translocation of mRNAs across the nuclear pore requires a protein carrier, which is part of a complex system located in the nuclear envelope. In this view, eIF-5A would be a part of a transport system that is involved in the translocation of nuclear RNA to the cytoplasm. After transit through the nuclear pores and assembly of translation-competent ribosomes, the preferentially translocated mRNAs would be utilized in the process of protein synthesis. Therefore, small molecular weight chemical substances that interfere with the biological function of the eIF-5A protein at the level of nuclear transport are potential antiviral compounds by interfering with nucleocytoplasmic translocation of viral mRNAs, especially in the field of HIV associated diseases.

The eIF-5A protein has a unique biochemical feature in that it is the only eukaryotic cellular protein known containing the unusual amino acid Hypusine. Hypusine formation is a modification of the lysine residue at amino acid position 50 within the 154 amino acids containing eIF-5A precursor molecule by a two-step posttranslational enzymatic reaction. In the first step, the cellular enzyme deoxyhypusine synthase (DHS) catalyzes the transfer of an aminobutyl-moiety from spermidine to the lysine 50 within eIF-5A via an enzyme-substrate intermediate formation at lysine 329 of the DHS to generate the eIF-5A deoxyhypusine form. This eIF-5A deoxyhypusine intermediate is subsequently hydroxylated by the enzyme deoxyhypusine hydroxylase, resulting in biologically active, hypusine-containing eIF-5A. Hypusine formation is essential for the biological activity of the eIF-5A protein. In addition, biologically active eIF-5A protein is essential for nuclear export of Rev proteins and, thereby, HIV replication, as well as for the function of the Human T-cell leukemia virus type I (HTLV-I) Rex protein which is itself crucial for HTLV-I replication.

### Hepatitis Viruses (HBV, HCV)

Hepatitis is an inflammation of the liver that is most often caused by infection with one of five viruses, hepatitis A, B, C, D or E. In cases, particularly in those related to hepatitis B and C, "chronic hepatitis" may result. Chronic hepatitis occurs when the body is unable to completely clear the virus even though the symptoms may not persist. Continued presence of the virus over a number of years can lead to cirrhosis (scarring of the liver) or hepatocellular carcinoma (liver cancer). It is estimated that approximately 350 million people worldwide are infected with chronic hepatitis viruses.

### Herpes simplex virus type 1 (HSV-1)

Herpes simplex virus is a highly adapted human pathogen with an extremely rapid lytic replication cycle, and yet with the ability to invade sensory neurons where highly restricted gene expression occurs with the absence of cytopathology. Such latent infections are subject to reactivation whereby infectious virus can be recovered in peripheral tissue enervated by the latently infected neurons following a specific physiological stress. Herpes simplex virus types 1 and 2 are generally associated with mucocutaneous facial infection (herpes labialis), genital infection (herpes genitalis), ophthalmic infection (herpes keratitis) and neonatal infection. In immunocompromised hosts, recurrence of HSV-1 may cause progressive fulminant lesions that are refractory to current antiviral therapy.

### Epstein-Barr virus (EBV)

Epstein-Barr virus is a member of the herpesvirus family and one of the most common human viruses. The virus occurs worldwide, and most people become infected with EBV sometime during their lives. When infection with EBV occurs during adolescence or young adulthood, it causes infectious mononucleosis 35% to 50% of the time. EBV also establishes a lifelong dormant infection in some cells of the body's immune system. A late event in a very few carriers of this virus is the emergence of Burkitt's lymphoma and nasopharyngeal carcinoma, two rare cancers that are not normally found in the United States. There is no specific treatment for infectious mononucleosis, other than treating the symptoms.

### Human herpesvirus 8 (HHV8), or Kaposi's sarcoma-associated herpesvirus (KSHV)

KSHV is a virus belonging to the family of herpesviruses. KSHV causes a blood vessel cancer called Kaposi's sarcoma (KS), a lymphoma (a cancer of the lymphocyte) called body cavity-based lymphoma and some forms of severe lymph node enlargement, called Castleman's disease. The real problem with KSHV occurs when an infected person becomes immunosuppressed. This occurs among transplant patients and patients receiving chemotherapy. There is no vaccine for KSHV.

### Varicella-zoster virus (VZV)

Chicken pox is an infection that is caused by the Varicella-zoster virus (VZV). VZV spreads in nasal discharge and in fluid from inside the chicken pox blisters. Chicken pox is very contagious, and 90% of people who are not immune will catch it when they are exposed. Epidemics are most common in the late winter and early spring, and children between ages 5 and 9 account for half of all cases.

Normally, chicken pox is a mild illness, but it can cause serious complications, including pneumonia, encephalitis, and serious bacterial infections of chicken pox blisters. After causing an attack of chicken pox, VZV remains in the body. It lies dormant in nerve cells and may be reactivated later in life to cause shingles.

### Respiratory syncytial virus (RSV)

RSV is a major cause of respiratory illness in young children. RSV infection produces a variety of signs and symptoms involving different areas of the respiratory tract, from the nose to the lungs.
In children younger than age three, RSV can cause a lower respiratory tract illness like bronchiolitis or pneumonia and may lead to respiratory failure.
Prevention and treatment: RespiGam is a vaccine used to protect infants who are considered to be at high risk for severe illness if they are infected with RSV, such as those with chronic lung diseases. This intravenous preventive treatment takes several hours to administer and must be repeated monthly during RSV season. A similar vaccine, Synagis, has become available more recently. It has replaced RespiGam as the treatment of choice for most high-risk children because it is given as monthly intramuscular injections, making it more convenient and less time-consuming to administer. Ribavirin is the only drug available, with only moderate efficacy.

### 2) TNFα-related inflammatory conditions and diseases

The main indications in this section comprise diseases like **Rheumatoid arthritis (RA), Adult (acute) Respiratory Distress Syndrome (ARDS), Intestinal Bowel Disease (IBD; Crohn's Disease), Psoriasis, Pancreatitis.**

Those indications constitute huge markets with unmet medical need. Inhibition of hypusine-formation on the eIF-5A protein results in the blockade of the nucleocytoplasmic transport of TNF-α mRNA and consequently in TNF-α formation. Therefore, inhibitors of hypusine formation like the ones of the present invention are considered as novel TNF-α nuclear transport inhibitors that may prove beneficial in the above mentioned diseases.
Surprisingly, it was found that the compounds of the general formula (I) and (II) exhibit excellent activity against formation of TNF-α.

### 3) Graft rejection after organ or tissue transplantations

Surprisingly, it was found that the compounds of the general formula (I) and (II) exhibit excellent activity against maturation of dendritic cells, a feature which is highly beneficial in preventing graft rejections following organ or tissue transplantation.

Dendritic cells (DC) are considered to be the most potent antigen-presenting cells, and CD83 is expressed at a high level on immune-competent, activated and mature DC. CD83 is a 45-kDa glycoprotein and member of the immunoglobulin (Ig) super-family. It is the best known marker for mature dendritic cells. Although the precise function of CD83 is not known, its selective expression and upregulation together with the costimulators CD80 and CD86 suggests an important role of CD83 in the induction of immune responses.

DC are potent antigen presenting cells that possess the unique ability to stimulate naive T-cells and are crucial in the induction of immunological responses. DC based immunotherapies are thus currently considered a particularly promising approach for cellular immunotherapy.

By studying DC derived from various tissues it has been shown that the morphology, phenotype and function of DC alter as they undergo a complex process of maturation. DC are derived from bone marrow progenitors and circulate in the blood as immature precursors prior to migration into the peripheral tissues. Within tissues DC are specialised in the taking up and processing of antigen so that it may be presented on MHC class II molecules. Upon appropriate stimulation, tissue DC undergo further maturation and migrate to secondary lymphoid tissue where they present antigen to T-cells and induce an immune response. Studies of DC maturation in vitro have defined the cytokines regulating their development from CD34+ myelomonocytic progenitors as well as from more mature peripheral blood precursors. An alternative pathway of differentiation from thymic precursors has also been described. As a result of these studies, DC may now be generated and manipulated ex-vivo for clinical applications in oncology, autoimmune disease and transplantation.
Following enrichment, DC develop an activated phenotype with up-regulation of CD80, CD86, and CD83 expression.
CD83 is a functionally important receptor that can regulate the development of cellular immunity by interacting with its ligand(s) .

Immature DCs capture antigens in the periphery but lack full T cell-stimulatory capacity. In the presence of appropriate stimuli (such as microbial products and/or inflammatory cytokines), the DCs then mature. DCs upregulate T cell adhesion and costimulatory molecules as well as selected chemokine receptors that guide DC migration into secondary lymphoid organs for priming of antigen-specific T cells. DCs are defined by their potent T cell-stimulatory capacity (e.g., in the allo-MLR) as well as a characteristic morphology (nonadherent cells with motile veils) and phenotype (upregulation of CD86 and de novo expression of CD83).

DC can be generated in vitro either from rare proliferating CD34⁺ or frequent, nonproliferating CD14⁺ monocytic precursors. The generation of DCs from monocytes under the use of GM-CSF and IL-4 yields homogenous DC progenitors that are well suited for studying the maturation of DC in vitro.

Functional, mature DC are derived from circulating precursor cells after a period of maturation and express then a specific array of marker molecules. These include the accessory/costimulatory gene products CD40, CD80, and CD86 as well as MHC class I and II. In particular, the presence of the CD83 molecule is a well characterized marker for fully mature DC, as CD83 can not be detected on immature DC precursors.

It is known from literature that formation of hypusine on the eukaryotic initiation factor 5A (eIF-5A) protein is required for the expression of the DC-specific molecule CD83 and the full stimulatory activity of mature DC. The hypusine formation is required for efficient nuclear export of CD83 mRNA. Thus, CD83 mRNA exploits a specific pathway for its transport from the nuclear site of RNA transcription and processing to the site of translation in the cytoplasm.

The eIF-5A protein has an unique biochemical feature in that it is the only eukaryotic cellular protein known containing the unusual amino acid hypusine. Hypusine formation is a modification of the lysine residue at amino acid position 50 within the 154 amino acids containing eIF-5A precursor molecule by a two-step posttranslational enzymatic reaction. In the first step, the cellular enzyme deoxyhypusine synthase (DHS) catalyzes the transfer of an aminobutyl-moiety from spermidine to the lysine 50 within eIF-5A via an enzyme-substrate intermediate formation at lysine 329 of the DHS to generate the eIF-5A deoxyhypusine form. This eIF-5A deoxyhypusine intermediate is subsequently hydroxylated by the enzyme deoxyhypusine hydroxylase, resulting in biologically active, hypusine-containing eIF-5A. Hypusine formation is essential for the biological activity of the eIF-5A protein. In addition, biologically active eIF-5A protein is essential for nuclear export of Rev proteins and, thereby, HIV replication, for the function of the Human T-cell leukemia virus type I (HTLV-I) Rex protein which is itself crucial for HTLV-I replication, as well as for the cell surface expression of the CD83 molecule which is crucially involved in the maturation of dendritic cells.

In general, the translocation of mRNAs across the nuclear pore requires a protein carrier, which is part of a complex system located in the nuclear envelope. In this view, eIF-5A would be a part of a transport system which is involved in the translocation of nuclear RNA to the cytoplasm. After transit through the nuclear pores and assembly of translation-competent ribosomes, the preferentially trans-located mRNAs would be utilized in the process of protein synthesis. Therefore, small molecular weight chemical substances that interfere with the biological function of the eIF-5A protein at the level of nuclear transport are potential compounds by interfering with nucleocytoplasmic translocation of mRNAs, especially in the field of organ transplantations.

The compounds of the present invention and/or pharmaceutically active salts thereof can be used for the manufacture of an agent especially especially for prophylaxis and prevention of graft rejections after organ or tissue transplantations. Examples for such organ transplantations comprise kidney, liver, heart, heart/lung, pancreas, corneas, bone marrow, skin, bone, heart valves, lung, intestine transplantations.

The compounds of the present invention act as inhibitors of dendritic cell maturation. Thus, a further aspect of the present invention is related to a method for inhibiting maturation of dendritic cells for the prevention or treatment of graft rejections after organ or tissue transplantations, comprising administering a subject in need thereof a pharmaceutically effective amount of at least one compound of the general formula (I) and/or (II) and/or pharmaceutically active salts thereof.

### 4) Cancer treatment

The present invention discloses the use of 3,5-Bis-(guanidiniminoethylphenyl)-guanidinoheptyl-amines and (2-Amino-1-methyl-pyridiniumchloride)-[3,5-bis-(guanidinimino-ethylphenyl]-N-guanidinoheptyl-amines as inhibitors of regu-lation of cellular hyperproliferation for the treatment of various cancer diseases. It was previously shown that the hypusine modification on the eIF-5A protein is a valuable novel target for anti-cancer therapy ( M.H. Park et al., Biofactors 1993, 4, 95-104). Compounds of the current invent-ion and/or pharmaceutically acceptable salts thereof are useful for the manufacture of a pharmaceutical formulation for prophylaxis and/or treatment of cancer. The cancer is selected from the group comprising breast, central nervous system, colon, gastric, lung, melanoma, head and neck, ovarian, cervix, glioblastoma, prostate, testis, leukaemia, liver, and renal cancer.

### DETAILED DESCRIPTION OF THE INVENTION

The present invention relates to compounds and methods for use of the general formula (I) and/or (II) and/or pharmaceutically acceptable salts thereof as inhibitors of nuclear transport or export of messenger RNAs of proteins involved in the biology of viral infections, TNF-dependent inflammatory diseases, organ rejections, and cancer.

The compounds of the present invention and/or pharmaceutically active salts thereof can be used for the manufacture of an agent for prophylaxis and/or treatment of viral diseases, TNF-dependent inflammatory conditions, graft rejection following organ transplantation, and cancer.
Surprisingly it was found that the compound of the present invention and pharmaceutically acceptable salts thereof are potent inhibitors of Hypusine formation on the eIF-5A protein. Thus, as the inhibition of Hypusine formation is a prerequisite for specific biological transport mechanisms, inhibition of specific nuclear transport pathways represents a clear molecular target for abovementioned disease situations.

Since eIF-5A protein is involved in the nuclear transport, the compounds of the present invention act as modulators of nuclear transport, particularly nuclear transport of RNA molecules. More particularly, the compounds of the inventions may be used for the manufacture of an agent capable of inhibiting the export of viral RNA molecules, TNF-alpha RNA molecules, and/or CD83 RNA molecules from the nucleus to the cytoplasm in cells of a person. Particularly, the present invention provides an composition and a method for the prevention or treatment of viral diseases, inflammatory diseases, graft rejections in organ transplantations, and cancer.

Thereby, the compounds of the invention and/or pharmaceutically acceptable salts thereof are administered in an ex-vivo dosage corresponding to an effective concentration in the range of 0.001-100µM. More preferably, the compounds of the present invention are administered in a dosage corresponding to an effective concentration in the range of 0.01-10µM. Most preferably, the compounds of the present invention are administered in a dosage corresponding to an effective concentration in the range of 0.1-5µM.

Furthermore, the compounds of the invention may be administered directly or in combination with further therapeutic compounds, especially with further agents used in viral diseases, inflammatory diseases, organ transplantations and cancer.
Thus, the compounds described in the present invention can be used in a monotherapy directly or in form of pharmaceutically acceptable compositions in order to treat viral diseases, inflammatory diseases, graft rejection in organ transplantations, and cancer.

The compounds of the general formulas (I) and (II) are basic and form pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogenbenzenesulfonic acid, picric acid, adipic acid, d-o-tolyltartraric acid, tartronic acid, α-toluic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.
The free base forms may be regenerated by treating the salt with a suitable dilute aqueous base solution such as dilute aqueous sodium hydroxide, potassium carbonate, ammonia and sodium bicarbonate. The free base forms differ from their corresponding salt forms somewhat in certain physical properties, such as solubility in polar solvents, but the salts are otherwise equivalent to their corresponding free base forms for purposes of this invention.

A still further aspect of the present invention relates to pharmaceutical compositions comprising at least one compound according to the invention and/or pharmaceutically acceptable salts thereof as an active ingredient and a pharmaceutically acceptable carrier, excipient, adjuvent and/or diluent.

The compounds of the general formulas (I) to (II) can also be administered in form of their pharmaceutically active salts optionally using substantially nontoxic pharmaceutically acceptable carrier, excipients, adjuvents or diluents. The medications of the present invention are prepared in a conventional solid or liquid carrier or diluent and a conventional pharmaceutically-made adjuvant at suitable dosage level in a known way. The preferred preparations are in administratable form which is suitable for oral application. These administratable forms, for example, include pills, tablets, film tablets, coated tablets, capsules, powders and deposits. Other than oral adminisratable forms are also possible. The inventive guanylhydrazone compounds of the general formulas (I) and/or (II) or pharmaceutical preparations containing said compounds may be administed by any appropriate means, including but not limited to injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrally, intracutanly, intravaginally, intravasally, intranasally, intrabuccally, percutanly, sublingually, or any other means available within the pharmaceutical arts.
Within the disclosed methods the pharmaceutical compositions of the present invention, containing at least one compound according to the invention or pharmaceutically acceptable salts thereof as an active ingredient will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.
Suitable binders include starch, gelatin, natural sugars, corn sweeteners, natural and synthetic gums such as acacia, sodium alginate, carboxymethyl-cellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.
Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.
Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.
Aerosol preparations suitable for inhalation may include solutions and solids in powder form, which may be in combination with a pharmaceutically acceptable carrier such as inert compressed gas, e.g. nitrogen.
For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.
Also included are solid form preparations which are intended to be converted, shortly before use, to liquid form preparations for either oral or parenteral administration. Such liquid forms include solutions, suspensions and emulsions.
The inventive compounds may also be deliverable transdermally. The transdermal compositions may take the form of creams, lotions, aerosols and/or emulsions and can be included in a transdermal patch of the matrix or reservoir type as are conventional in the art for this purpose.
The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.
Tablet means compressed or molded solid dosage form containing the active ingredients with suitable diluents. The tablet can be prepared by compression of mixtures or granulations obtained by wet granulation, dry granulation or by compaction well known to a person skilled in the art.
Oral gels refers to the active ingredients dispersed or solubilized in a hydrophillic semi-solid matrix. Powders for constitution refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluent in the composition can range from about 5 to about 95% by weight of the total composition, preferably from about 25 to about 75%, more preferably from about 30 to about 60% by weight.
The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 2 to about 20% by weight of the composition, more preferably from about 5 to about 10% by weight.
Binders characterize substances that bind or "glue" powders together and make them cohesive by forming granules, thus serving as the "adhesive" in the formulation. Binders add cohesive strength already available in the diluent or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropylmethylcellulose; polyvinylpyrrolidone; and inorganics such as magnesium aluminum silicate. The amount of binder in the composition can range from about 2 to about 20% by weight of the composition, more preferably from about 3 to about 10% by weight, even more preferably from about 3 to about 6% by weight.
Lubricant refers to a substance added to the dosage form to enable the tablet, granules, etc. after It has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'l-leucine. Lubricants are usually added at the very last step before compression, since they must be present on the surfaces of the granules and in between them and the parts of the tablet press. The amount of lubricant in the composition can range from about 0.2 to about 5% by weight of the composition, preferably from about 0.5 to about 2%, more preferably from about 0.3 to about 1.5% by weight.
Glidents are materials that prevent caking and improve the flow characteristics of granulations, so that flow is smooth and uniform. Suitable glidents include silicon dioxide and talc. The amount of glident in the composition can range from about 0.1% to about 5% by weight of the total composition, preferably from about 0.5 to about 2% by weight.
Coloring agents are excipients that provide coloration to the composition or the dosage form. Such excipients can include food grade dyes and food grade dyes adsorbed onto a suitable adsorbent such as clay or aluminum oxide. The amount of the colouring agent can vary from about 0.1 to about 5% by weight of the composition, preferably from about 0.1 to about 1%.

Techniques for the formulation and administration of the compounds of the present invention may be found in "Remington's Pharmaceutical Sciences" Mack Publishing Co., Easton PA. A suitable composition comprising at least one compound of the invention may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

The synthesis and use of the innovative compounds is described. The invention further encompasses screening assays to test additional compounds for the above-mentioned activities, and pharmaceutical compositions useful in the practice of this method in therapy.

### Examples

### Compounds and their synthesis

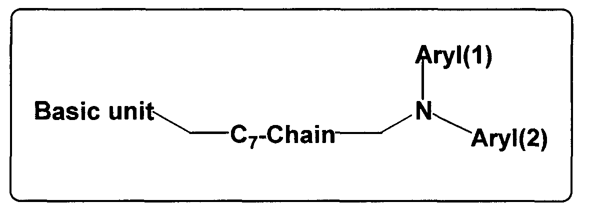

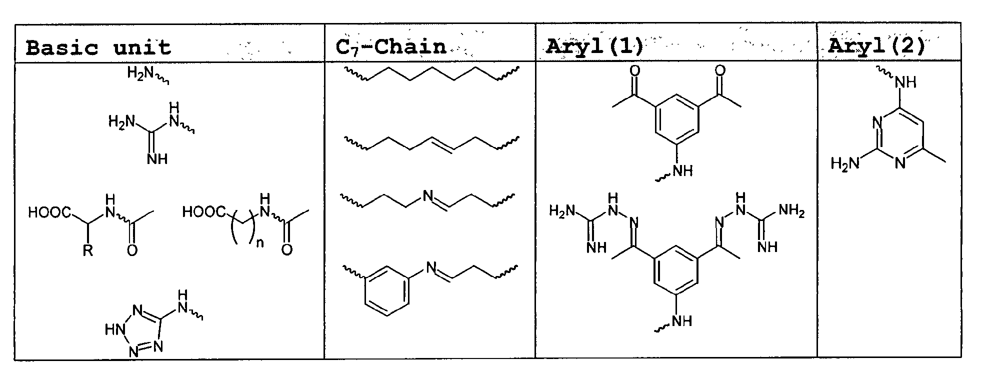

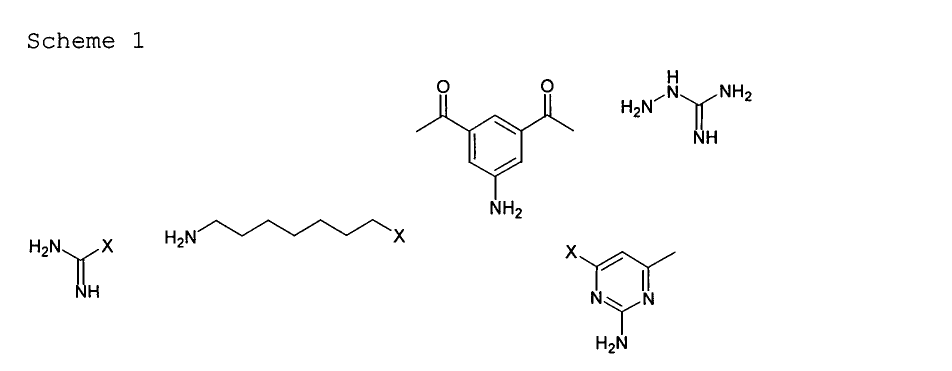

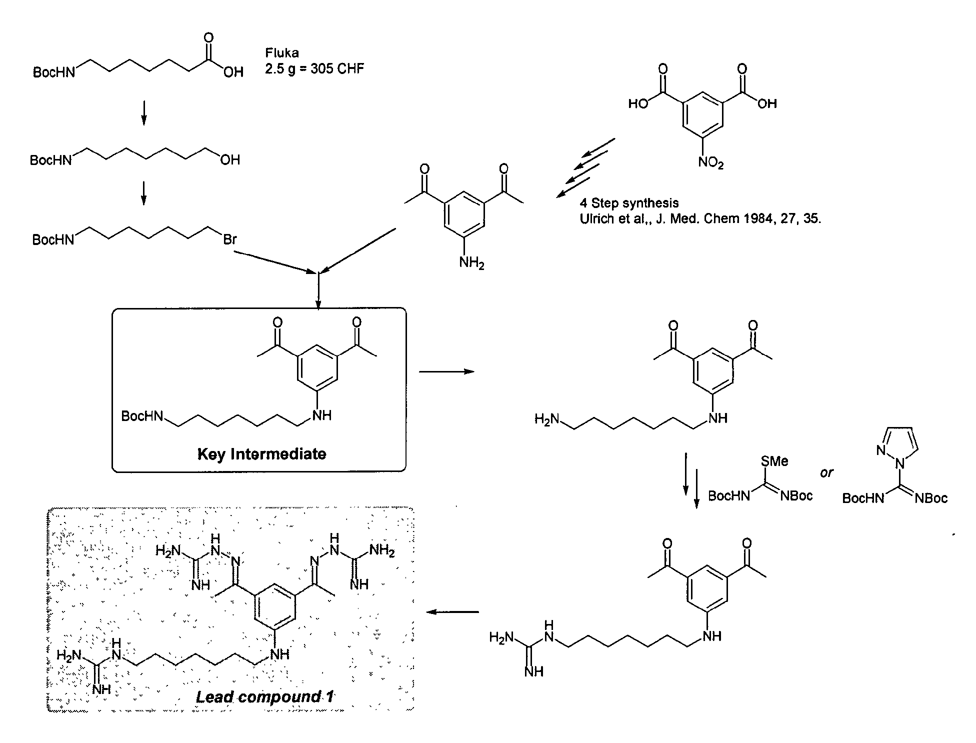

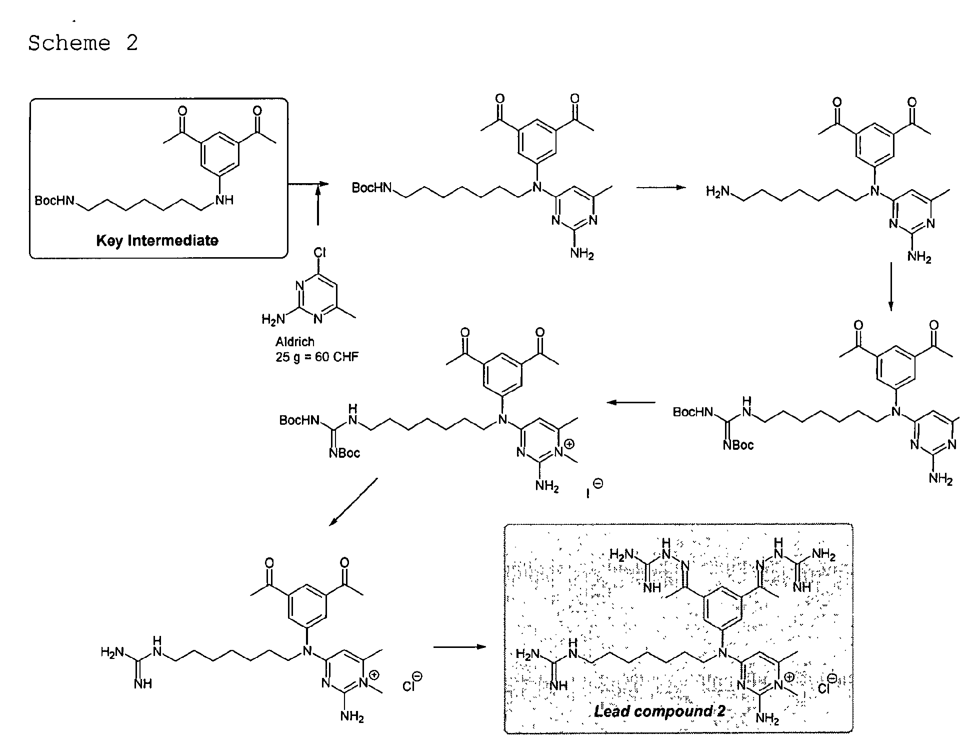

### Materials and methods

### HIV detection

HIV replication was measured either by p24 HIV Antigen Capture Assay Kit (ELISA; HIVAG-1 Monoclonal kit B1A011; Abbott) following the manufacturer's recommendation, or by using the Quantiplex HIV-1 RNA 3.0 Assay (bDNA) system (Chiron Diagnostics). The latter assay is a signal amplification nucleic acid probe assay (employing a sandwich nucleic acid hybridization procedure) for direct quantitation of HIV RNA in human plasma. There, in brief, HIV-1 is first concentrated from plasma by centrifugation. After release of genomic HIV-1 RNA from the virions, the RNA is captured to a microwell by a set of specific, synthetic oligonucleotide capture probes. A set of target probes hybridize to both the viral RNA and the pre-amplifier probes. The capture probes, comprised of 17 individual capture extenders, and the target probes, comprised of 81 individual target extenders, bind to different regions of the pol gene of the viral RNA. The amplifier probe hybridizes to the pre-amplifier forming a branched DNA (bDNA) complex. Then, multiple copies of an alkaline phosphatase labeled probe are hybridized to this immobilized bDNA complex. Detection is achieved by incubating the entire complex with a chemoluminescent substrate. Light emission is directly proportional to the amount of HIV-1 RNA present in each sample, and results are recorded as relative light units by an appropriate analyzer.

### Cells and HIV strains

Jurkat cells (species: human T-cell leukemia; special characteristics: cells are permissive for growth of T-cell tropic HIV strains; source:ATCC Cat.No.TIB 152) or PM1 cells (species: clonal derivative of human HUT 78 cells; special characteristics: cells are permissive for growth of macrophagetropic and T-cell tropic HIV strains; source: Dr. Marvin Reitz, courtesy of the NIH AIDS Research and Reference Reagent Program Cat.No. 3038) were stimulated with Phytohemagglutinin (PHA-P, Product No. L9132 from Sigma) in a concentration of 2 µg/ml (1.5x10⁶ cells/ml) and Hexadimethrine Bromide (Polybrene, Product No. H9268 from Sigma) in a concentration of 2 µg/ml (1.5 x 10⁶ cells/ml) in RPMI 1640 medium containing 10 % fetal calf serum (FCS, Pansystems GmbH) and antibiotics for 3 days.

For HIV-1 infection, 5x10⁷ cells were resuspended in 500 µl culture medium without test drugs and incubated in a 50 ml blue-cap-tube at 37°C for 5 hrs with HIV-1 high titer viral stocks. Jurkat cells were infected with the T-cell tropic strain HIV-1 NL4-3, PM1 cells with the macrophage tropic strain HIV-1 Ba-L (both from the NIH AIDS Research and Reference Reagent Program). After infection, cells were washed twice with PBS without Ca²⁺ and Mg²⁺ to avoid false positive p24 antigen determination. Cells were resuspended, and identical aliquots (1x10⁶/ml) of infected cells were further cultured in 10 ml medium with test drugs at various concentrations, or in medium with DMSO as control for calculation of the inhibition of virus replication [in %].

Culture medium was changed and cells were split at days 3 and 7 of the experiments. Viability of the cells (Trypan-blue staining), cell counts and p24 antigen levels were determined at days 3, 7 and 10 of the experiments. No significant differences in cell viability were observed (∼85-90 % living cells).

### HBV Experiments

Human HepG2-2.2.15 cells (ATCC Cat. No. CRL-11997), chronically infected with HBV, are plated in 96-well microtiter plates in DMEM medium supplemented with fetal bovine serum. After incubation at 37°C in a humidified, 5% CO₂ environment for 16-24 hours, the monolayer of HepG2-2.2.15 cells is washed and the medium is replaced with complete medium containing various concentrations of test compound. Every three days during the experiments, the culture medium is replaced with fresh medium containing the appropriately diluted test compound. Six days following the initial administration of the test compound, the cell culture supernatant is collected and clarified by centrifugation. The cell viability is evaluated by a dye uptake procedure (Promega's Cell Titer Aqueous One Solution). Virion-associated HBV DNA present in the tissue culture supernatant is amplified employing PCR technology using primer pairs derived from the HBV strain ayw. Subsequently, the PCR-amplified HBV DNA is detected in real-time by monitoring increases in fluorescence signals that result from exonucleolytic degradation of a quenched fluorescent probe molecule following hybridization of the probe to the amplified HBV DNA (TaqMan; Perkin-Elmer).
The same type of experiments was additionally performed with a mutant, Lamivudine-resistant HBV strain, which was transfected into HepG2 cells.

### Herpes simplex virus type 1 experiments:

A virus-induced cytopathic effects (CPE)-inhibition assay procedure using Promega's Cell Titer Aqueous One Solution (MTS, metabolic dye) is employed to evaluate compounds for antiviral activity against herpes simplex virus type 1 (strain HF) in Vero cells (African green monkey cells, ATCC Cat. No. CCL-81). Vero cells are pregrown in 96-well tissue culture plates using Dulbecco's Modified Eagle's Medium (DMEM) supplemented with 10% heat-inactivated fetal bovine serum (FBS), L-glutamine, penicillin, and streptomycin. To each of the replicate cell cultures is added 50 µl of the test drug solution and 50 µl of virus suspension. Cell controls containing medium alone, virus-infected controls containing medium and virus, drug cytotoxicity controls containing medium and each drug concentration, reagent controls containing culture medium only (no cells), and drug colorimetric controls containing drug and medium (no cells) are run simultaneously with the test samples. The plates are incubated at 37°C in a humidified atmosphere containing 5% CO₂ until maximum CPE is observed in the untreated virus control cultures (Day 5). CPE inhibition is determined by a dye (MTS) uptake procedure. This method measures cell viability and is based on the reduction of the tetrazolium MTS by mitochondrial enzymes of viable host cells to MTS formazan. MTS (10 µl) is added to each of the plate wells. The plates are incubated at 37°C for 4 hours. The purple color of the MTS formazan is then measured spectrophotometrically at 490/650 nm. The optical density (OD) value of each culture is a function of the amount of formazan produced which is proportional to the number of viable cells. A computer program is utilized to calculate the percent of CPE reduction of the virus-infected wells and the % cell viability of uninfected drug control wells.

### Epstein-Barr virus experiments:

Human P3HR-1 cells are latently infected with EBV. Culturing these cells in the presence of phorbol ester induces a lytic infection cycle, resulting in stimulation of viral replication within the cells. Compound efficacy in this assay is determined using TaqMan PCR technology, and compound toxicity is measured by addition of Promega's Cell Titer Aqueous One Solution (metabolic dye).

### Assay Set-Up

1. P3HR-1 cells are counted by the trypan blue dye exclusion method, and the cell count/ml is determined. Cells are pelleted by centrifugation and washed once with PBS to minimize the basal level of virus released from untreated cells which spontaneously enter the lytic cycle and release virus.
2. Cells are resuspended in fresh media and adjusted to the appropriate cell density. Cells are then plated in the interior wells of 96-well round-bottom microtiter plates in a volume of 50 µl per well.
3. The phorbol ester TPA is added to appropriate wells in a volume of 50 µl per well to yield a final concentration of 15 ng/ml. Medium is added in place of TPA in three wells to serve as a negative control.
4. Test compounds are diluted in DMSO to a stock concentration which is 400x the desired high test concentration. These compounds are further diluted in complete medium in 1.2 ml titertubes to yield a solution which is 2x the desired high test concentration. Serial half-log dilutions of this solution are performed in complete medium to yield a total of 6 test concentrations for each test compound. Compound dilutions are added to appropriate wells of the microtiter plate in a volume of 100 µl per well. Medium containing no test compound is added to the virus control and cell control wells. In addition, 200 µl of medium containing no test compound is added to the exterior wells of the plate.
5. Plates are incubated at 37°C in a humidified CO₂ incubator until day 4 post drug addition.
6. Duplicate plates are set up plating media in place of TPA for evaluation of compound toxicity.

### Determination of Compound Efficacy and Toxicity

1. Efficacy plates are removed from the incubator, freeze thawed twice and cell lysate samples (100 µl) are removed from each well and transferred into 96-well storage plates.
2. Pronase is added to the supernatant samples to a final concentration of 0.75 mg/ml. Samples are incubated at 37°C for 30 minutes. Samples are then treated with 1 Unit of DNase per well and incubated at 37°C for 60 minutes. Encapsidated DNA from intact virions is not affected by this treatment. DNase is inactivated by heating samples to 95°C for 15 minutes.
3. PCR reaction mixtures are prepared from reagents provided in the PE Applied Biosystems TaqMan PCR Reagent Kit according to the manufacturer's directions. Total reaction volumes are 50 µl each. Master reaction mix is dispensed into optical PCR tubes in a volume of 47 µl. Samples (3 µl) are added to the reaction mix and mixed thoroughly.
4. Reaction plates are loaded into a PE Applied Biosystems 7700 Sequence Detector, and a run cycle is initiated using the manufacturer's recommended PCR conditions.
5. A standard curve prepared from known copy numbers of DNA isolated from P3HR-1 cells and amplified is run with each plate in order to quantitate the DNA copy number in each original sample. The efficacy of the compound is determined by comparing DNA copy numbers from test wells with those of control wells.
6. Twenty micorliters of Cell Titer Aqueous One Solution (Promega) is added to each well of the toxicity plates. Plates are incubated at 37°C in a humidified CO₂ incubator for 4 hours or until sufficient color development has occurred. Plates are read on a VMax microtiter plate reader at a wavelength of 490/650 nm. Toxicity of the test compound is determined by comparing the optical density of test wells with that of control wells.

### Human Herpesvirus 8 experiments:

The human BCBL-1 cell line is available through the AIDS Reference and Reagent Program (Cat.No. 3233). This cell line was derived from a body cavity-based lymphoma latently infected with HHV-8. Culturing these cells in the presence of phorbol ester induces a lytic infection cycle, resulting in the release of viral particles into the medium. Compound efficacy in this proposed assay is determined using TaqMan PCR technology, and compound toxicity is measured by addition of Promega's Cell Titer Aqueous One Solution (metabolic dye).

### Assay Set-Up

7. BCBL-1 cells will be counted by the trypan blue dye exclusion method, and the cell count/ml will be determined. Cells will be pelleted by centrifugation and washed once with PBS to minimize the basal level of virus released from untreated cells which spontaneously enter the lytic cycle and release virus.
8. Cells will be resuspended in fresh media and adjusted to the appropriate cell density. Cells will then be plated in the interior wells of 96-well round-bottom microtiter plates in a volume of 50 µl per well.
9. The phorbol ester TPA will be added to appropriate wells in a volume of 50 µl per well to yield a final concentration of 15 ng/ml. Medium will be added in place of TPA in three wells to serve as a negative control.
10. Test compounds will be diluted in DMSO to a stock concentration which is 400x the desired high test concentration. These compounds will be further diluted in complete medium in 1.2 ml titertubes to yield a solution which is 2x the desired high test concentration. Serial half-log dilutions of this solution will be performed in complete medium to yield a total of 6 test concentrations for each test compound. Compound dilutions will be added to appropriate wells of the microtiter plate in a volume of 100 µl per well. Medium containing no test compound will be added to the virus control and cell control wells. In addition, 200 µl of medium containing no test compound will be added to the exterior wells of the plate.
11. Plates will be incubated at 37°C in a humidified CO₂ incubator until day 4 post drug addition.
12. Duplicate plates will be set up plating media in place of TPA for evaluation of compound toxicity.

### Determination of Compound Efficacy and Toxicity

7. Efficacy plates will be removed from the incubator, and supernatant samples (100 µl) will be removed from each well and transferred into 96-well storage plates.
8. Pronase will be added to the supernatant samples to a final concentration of 0.75 mg/ml. Samples will be incubated at 37°C for 30 minutes. Supernatant samples will then be treated with 1 Unit of DNase per well and incubated at 37°C for 60 minutes to degrade DNA which has been released by dead cells. Encapsidated DNA from intact virions will not be affected by this treatment. DNase is inactivated by heating samples to 95°C for 15 minutes.
9. PCR reaction mixtures well be prepared from reagents provided in the PE Applied Biosystems TaqMan PCR Reagent Kit according to the manufacturer's directions. Total reaction volumes will be 50 µl. Master reaction mix will be dispensed into optical PCR tubes in a volume of 47 µl. Samples (3 µl) will be added to the reaction mix and mixed thoroughly.
10. Reaction plates will be loaded into a PE Applied Biosystems 7700 Sequence Detector, and a run cycle will be initiated using the manufacturer's recommended PCR conditions.
11. A standard curve prepared from known copy numbers of DNA isolated from BCBL-1 cells and amplified will be run with each plate in order to quantitate the DNA copy number in each original sample. The efficacy of the compound will be determined by comparing DNA copy numbers from test wells with those of control wells.
12. Twenty microliters of Cell Titer Aqueous One Solution (Promega) will be added to each well of the toxicity plates. Plates will be incubated at 37°C in a humidified CO₂ incubator for 4 hours or until sufficient color development has occurred. Plates will be read on a VMax microtiter plate reader at a wavelength of 490/650 nm. Toxicity of the test compound will be determined by comparing the optical density of test wells with that of control wells.

### Varicella-zoster virus experiments:

MRC-5 (human embryonic lung fibroblast) cells (ATCC Cat. No. CCL-171) are seeded in 24 well plates using Eagle's Minimum Essential Medium (EMEM) supplemented with 10% heat-inactivated fetal bovine serum (FBS), L-glutamine, sodium pyruvate, non-essential amino acids, penicillin, and streptomycin. The plates are then incubated overnight at 37°C and 5% CO₂. The following day, media is aspirated and approximately 50-100 pfu (plaque forming units) of VZV strain ELLEN is added to the wells of each plate in a volume of 200 µl of assay media (EMEM supplemented with 5% heat-inactivated FBS, L-glutamine, sodium pyruvate, non-essential amino acids, penicillin, and streptomycin). The remaining wells of each plate serve as cellular control wells and receive 200 µl of assay media without virus. The virus is allowed to adsorb onto the cells for 1 hr at 37°C and 5% CO₂. Two dilutions of each test drug are prepared by diluting them in assay media containing 0.5% Methylcellulose. After the incubation period, 1 ml of each drug dilution is added to triplicate wells of a plate. Assay media (without drug) containing 0.5% Methylcellulose is added to the three cell control wells and to three virus control wells on each plate. The plates are incubated for 9-12 days to allow for plaque formation. The media is then aspirated from the wells and the cells are fixed and stained using 20% Methanol containing Crystal Violet. Plaques are enumerated by microscopic inspection and data is plotted as percent of virus contol.

Drug toxicity is determined in parallel by seeding MRC-5 cells in 96 well plates and adding drugs to triplicate wells at two dilutions. After a six day incubation period, cell viability is determined using CellTiter 96 Aqueous One Solution (Promega).

### Respiratory syncytial virus experiments:

A cytoprotection assay procedure using MTS is employed to evaluate compounds for antiviral activity against Respiratory Syncytial Virus (Long) in Vero (African green monkey, ATCC Cat. No. CCL-81) cells. The cells are pre-grown overnight at 37°C (2x10⁴/well in 96 well tissue culture plates) with 10% fetal Bovine serum containing Minimum Essential Medium. The positive control for the assay is Interferon-Gamma/Ribavirin. The infection medium contains reduced serum levels (2% FBS). Using half log dilutions with high test starting at 100 µg/ml the drug dilutions are made and added to triplicate cultures in 0.1 ml volume and pre-titrated virus suspension is also added in 0.1 ml volume. Cell control containing medium alone, virus controls containing medium and virus, the drug toxicity controls containing medium and each drug concentrations are run simultaneously with the test samples. The plates are incubated at 37C° with 5% CO₂ until maximum CPE (cytopathogenic effect) observed in untreated virus controls. CPE inhibition is determined by dye uptake procedure (Celltiter- MTS). Celltiter reagent contains a novel tetrazolium (MTS) compound and an electron coupling reagent phenazine ethosulfate (PES). MTS-tetrazolium is bioreduced by cells into a colored formazan product that is soluble in tissue culture medium. This conversion is accomplished by NADPH or NADH produced by dehydrogenase enzymes in metabolically active cells. The amount of soluble formazan produced by cellular reduction of the MTS is measured by the absorbance at 490 nm. The quantity of formazan product as measured by the amount of 490 nm absorbance is directly proportional to the number of living cells in the culture thus allowing the rapid quantitative analysis of the inhibition of virus-induced cell killing by the test substances.

### Dendritic Cell Experiments

Cells were cultured using a standard medium (referred to as 1% human plasma medium), which consisted of RPMI 1640 (BioWhittaker) supplemented with glutamine (300 µg/ml) (BioWhittaker), penicillin/streptomycin (20 µg/ml), 10 mM Hepes, pH 7.5 (Sigma-Aldrich) and 1% heat-inactivated (56°C; 30 min) human plasma from a single donor.

### Generation of DC

Peripheral Blood Mononuclear Cells (5 x 10⁷) were isolated from buffy coats by sedimentation in Ficoll-hypaque (Amersham) and seeded onto IgG-coated (10 µg/ml γ-globulin from Cohn fraction; Sigma-Aldrich) 100 mm-culture dishes and incubated at 37°C in 5% CO₂. After 1 and 7 h of incubation, nonadherent cell fractions were harvested, and the remaining adherent cells (predominantly monocytes) were further cultured in 1% human plasma medium supplemented with the cytokines GM-CSF (800 U/ml) and IL-4 (1,000 U/ml). Fresh medium (5 ml) containing 4,000 U GM-CSF and 5,000 U IL-4 was added to the culture dish at day 3 of this incubation period. On day 4 or 5, nonadherent cells were collected, counted, and transferred into new dishes at a density of 0.3-0.5 x 10⁵ cells/ml. For final DC maturation, 1% human plasma medium was supplemented with TNF-α (25 ng/ml), prostaglandin E₂ (PGE₂; 1 ng/ml), GM-CSF (400 U/ml), and IL-4 (500 U/ml).

### Cytokines

Recombinant human (rh)GM-CSFwas obtained from Novartis Research Institute, rhIL-4 from Genzyme, rhTNF-α from Boehringer, and PGE₂ from Cayman Chemical.

### Experimental Readouts

a) Uptake of dextran as a measure of dendritic cell activity
b) Expression of cell surface markers as a measure for dendritic cell phenotype. For flow cytometry analyses, mAbs recognizing the following antigens were used: CD83 (Immunotech), CD86, CD32, MHC class II (Becton Dickinson). The isotype controls IgG1a and IgG2b were obtained from Becton Dickinson and were run in parallel. Cell populations were analyzed on a FACScan™ (Becton Dickinson) according to the manufacturer's recommendations.
c) Antigen presentation to the T-lymphocytes in an allogeneic mixed lymphocyte reaction as a measure for the immunocompetence of the dendritic cells.

### Cell proliferation assays for cancer cells

To quantify cell proliferation, cellular metabolic activity is determined by oxidation of the tetrazoilium salt WST-1 (Roche, Cat.No. 1 644 807) and subsequent photometric analysis. Human tumor cells are cultivated either directly in 96 well plates or in tissue culter plates for longer compound exposure. Cells are incubated with different concentrations of test compounds for 3 or 11 days. Subsequently, 10 microl WST1 is added and mixtures are incubated for an additional 1 h. The amount of converted formazan salt is quantified at least in four replicates by photometric analysis at 450 versus 655 nm.

## Claims

1. A compound having the formula: 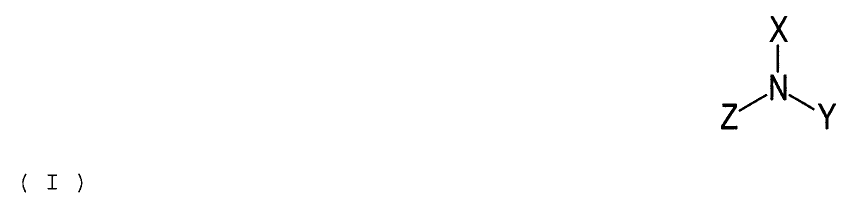 wherein:
X is independently of each other 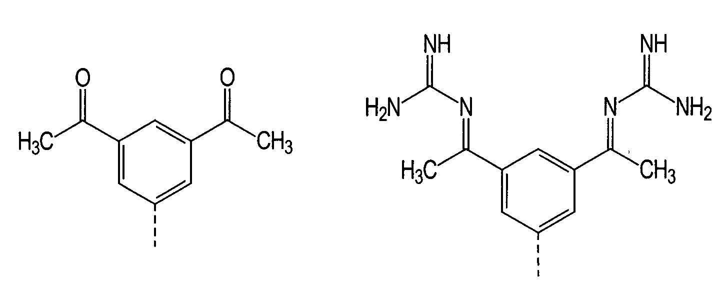
Y is independently of each other 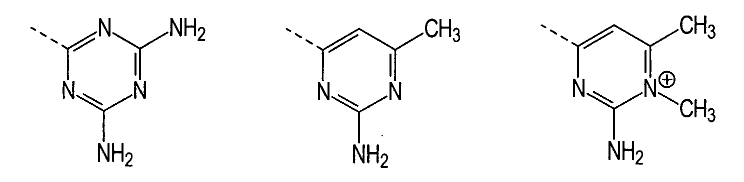
Z is independently of each other 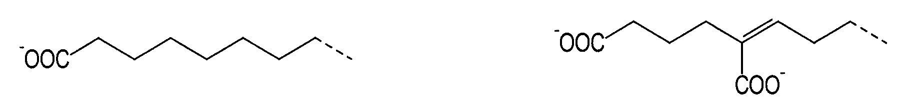 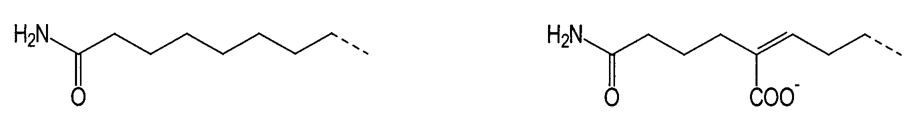 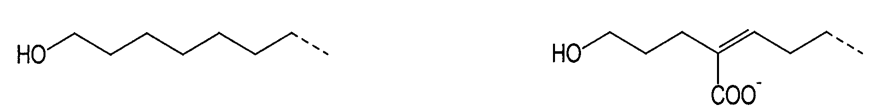 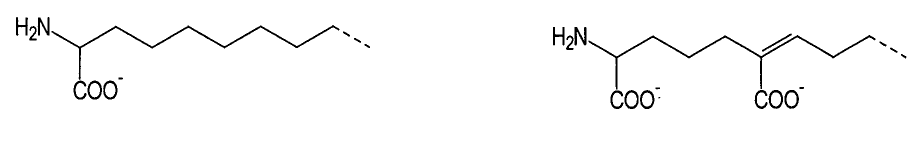
or to a compound selected form the following general formulas (II) 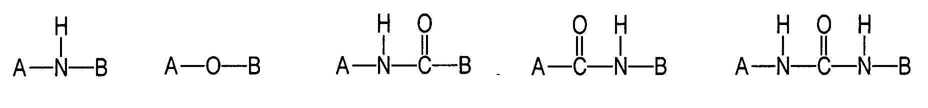 wherein
A is independently of each other 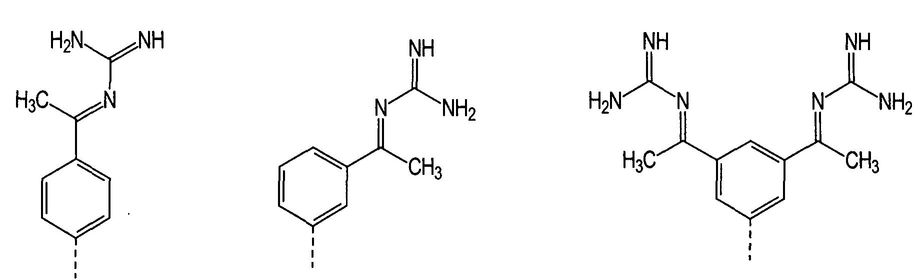 and
B is independently of each other 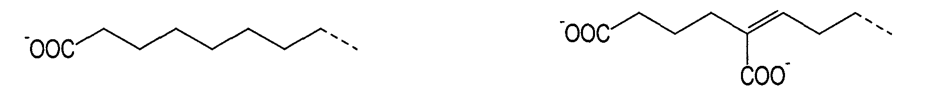 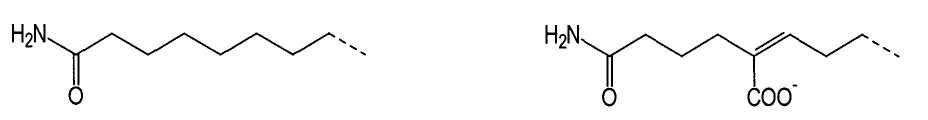 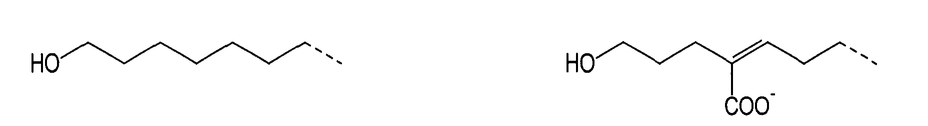 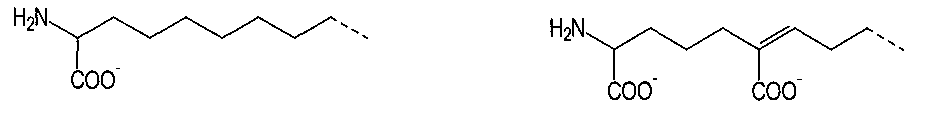 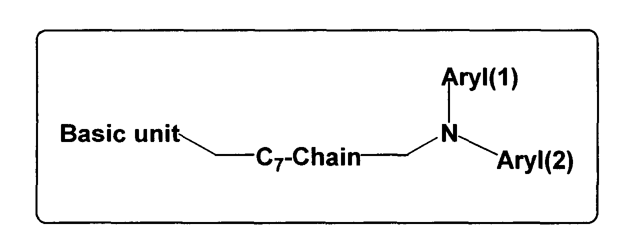 and pharmaceutically acceptable salts thereof.

2. The compound *N*-{7-[3,5-Bis-(1-guanidiniminoethyl)-phenylamino]-heptyl}-guanidine or *N*-[3,5-Bis-(1-guanidiniminoethyl)-phenyl]-*N*-(7-guanidinoheptyl)-amine or a salt thereof.

3. The compound termed *N*-(7-{(2-Amino-1-methyl-pyridiniumchloride-4-yl)-[3,5-bis-(1-guanidiniminoethyl)-phenylamino]-heptyl}-guanidine or *N*-(2-Amino-1-methyl-pyridiniumchloride-4-yl)-*N*-[3,5-bis-(1-guanidiniminoethyl)-phenyl]-*N*-(7-guanidinoheptyl)-amine or a salt thereof.

4. The lead compound 1 or a salt thereof.

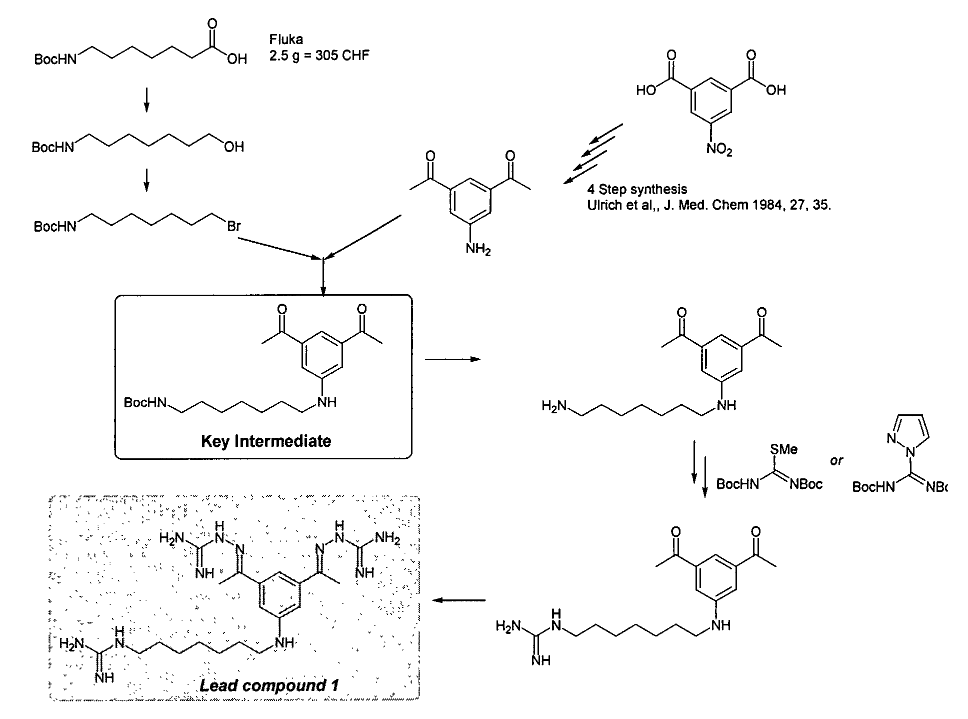

5. The lead compound 2 or a salt thereof.

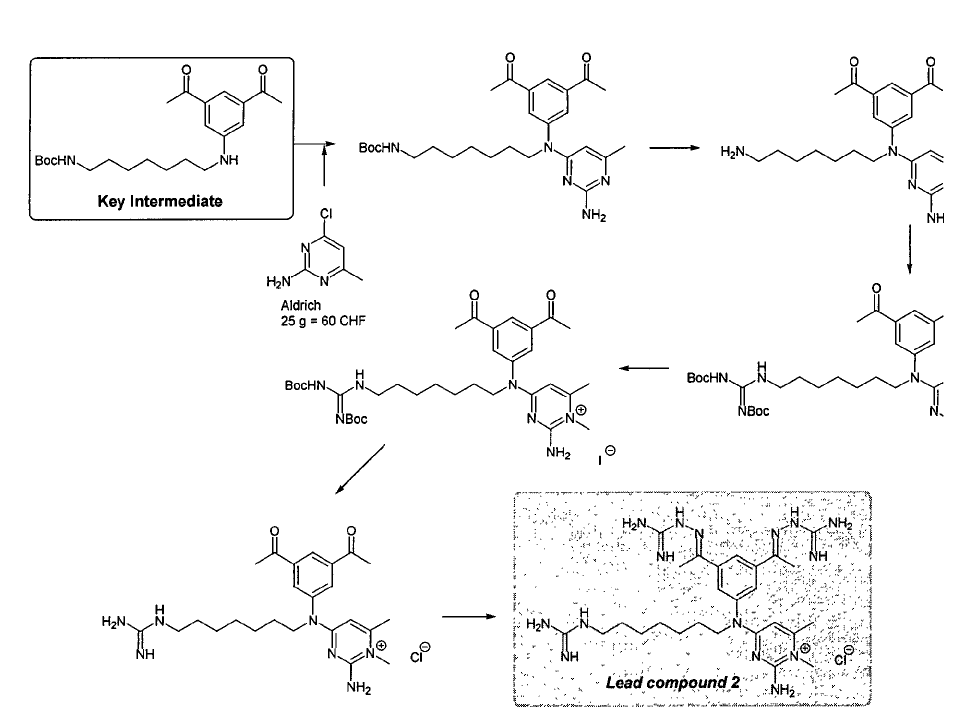

6. The pharmaceutical composition comprising the compound of claim 1 and a pharmaceutically acceptable carrier.

7. The pharmaceutical composition comprising the compound of claim 2 and a pharmaceutically acceptable carrier.

8. The pharmaceutical composition comprising the compound of claim 3 and a pharmaceutically acceptable carrier.

9. The pharmaceutical composition comprising the compound of claim 4 and a pharmaceutically acceptable carrier.

10. The pharmaceutical composition comprising the compound of claim 5 and a pharmaceutically acceptable carrier.

11. Use of a compound of the claims 1-10 as inhibitors of Hypusine formation.

12. Use of a compound of the claims 1-10 as an inhibitor of nuclear transport.

13. Use of the compounds from claims 1-10 and pharmaceutically acceptable salts thereof as pharmaceutically active agents for prophylaxis and/or treatment of virally induced infections and diseases, including opportunistic infections.

14. Use according to claim 13 wherein the virus is selected from retroviruses, adenoviruses, hepadnaviruses, herpesviruses, and influenza viruses and paramyxoviruses.

15. Use of the compounds from claims 1-10 and pharmaceutically acceptable salts thereof as pharmaceutically active agents for prophylaxis and/or treatment subjects in need of amelioration of the deleterious effects of tumor necrosis factor alpha production.

16. Use of the compounds from claims 1-10 and pharmaceutically acceptable salts thereof as pharmaceutically active agents for the manufacture of a medicament for the inhibition of a CD83 functionally dependent process in an individual.

17. Use according to claim 1-10 and 12, wherein said effect is the inhibition of the nucleocytoplasmic translocation of CD83 mRNA molecules.

18. Use according to the claim 17, wherein said inhibition is directed to the expression of CD83 protein.

19. Use according to claim 18, wherein the expression of CD83 on dendritic cells is inhibited.

20. Use according to claims 16-19, wherein said inhibition is directed to the maturation of dendritic cells.

21. Use according to any of the claims 16 to 20, for the treatment and / or prevention of graft rejection during or following organ transplantation.

22. Use according to claim 21, wherein the organ suitable for said transplantation is selected from the group: kidney, liver, heart, pancreas, lung and bone marrow.

23. Use according to any of the claims 16 to 22, wherein said treatment is achieved in combination with further medicaments used in or after organ transplantation.

24. Use according to the claims 1-10, and pharmaceutically acceptable salts thereof as pharmaceutically active agents for prophylaxis and/or treatment of cancer.

25. Use according to claim 24 wherein the cancer is selected from the group comprising breast, central nervous system, colon, gastric, lung, melanoma, head and neck, ovarian, cevix, glioblastoma, prostate, testis, leukemia, liver, and renal cancer.

26. Use of a compounds of any of the claims 1-10 and/or pharmaceutically acceptable salts thereof as an inhibitor of hyperproliferation of cancer cells.

27. Use according to any of the previous claims wherein the compound of the claims 1-10 or pharmaceutically acceptable salts thereof is administered in a dosage corresponding to an effective concentration in the range of 0.001-100 µM.

28. Use according to the claim 27 wherein the compound of the claims 1-10 or pharmaceutically acceptable salts thereof is administered in a dosage corresponding to an effective concentration in the range of 0.01-10 µM.

29. Method according to the claims 1-10 for inhibiting Hypusine formation.

30. Method according to the claims 1-10 for inhibiting nuclear transport.

31. Method according to the claims 1-10 for prophylaxis and/or treatment of virally induced infections and diseases, including opportunistic infections.

32. Method according to claim 13 wherein the virus is selected from retroviruses, adenoviruses, hepadnaviruses, herpesviruses, and influenza viruses and paramyxoviruses.

33. Method according to the 1-10 for prophylaxis and/or treatment subjects in need of amelioration of the deleterious effects of tumor necrosis factor alpha production.

34. Method according to claims 1-10 for the manufacture of a medicament for the inhibition of a CD83 functionally dependent process in an individual.

35. Method according to claim 1-10 and 12, wherein said effect is the inhibition of the nucleocytoplasmic translocation of CD83 mRNA molecules.

36. Method according to claim 17, wherein said inhibition is directed to the expression of CD83 protein.

37. Method according to claim 18, wherein the expression of CD83 on dendritic cells is inhibited.

38. Method according to claims 16-19, wherein said inhibition is directed to the maturation of dendritic cells.

39. Method according to any of the claims 16 to 20, for the treatment and / or prevention of graft rejection during or following organ transplantation.

40. Method according to claim 21, wherein the organ suitable for said transplantation is selected from the group: kidney, liver, heart, pancreas, lung and bone marrow.

41. Method according to any of the claims 16 to 22, wherein said treatment is achieved in combination with further medicaments used in or after organ transplantation.

42. Method according to the claims 1-10, for prophylaxis and/or treatment of cancer.

43. Method according to claim 24 wherein the cancer is selected from the group comprising breast, central nervous system, colon, gastric, lung, melanoma, head and neck, ovarian, cevix, glioblastoma, prostate, testis, leukemia, liver, and renal cancer.

44. Method according to the claims 1-10 for inhibition of hyperproliferation of cancer cells.

45. Method according to any of the previous claims wherein the compound of the claims 1-10 or pharmaceutically acceptable salts thereof is administered in a dosage corresponding to an effective concentration in the range of 0.001-100 µM.

46. Method according to the claim 45 wherein the compound of the claims 1-10 or pharmaceutically acceptable salts thereof is administered in a dosage corresponding to an effective concentration in the range of 0.01-10 µM.
